# EUROPEAN PATENT APPLICATION

(11) **EP 4 548 922 A1**
(43) Date of publication of application: **07.05.2025**
(21) Application number: 23831570.9
(22) Date of filing: 29.06.2023
(51) Int. Cl.: A61K 31/519, A61P 35/00, A61P 35/04

(54) **COMPOSITION FOR INHIBITING PROLIFERATION OF CANCER STEM CELLS**

(30) Priority: 30.06.2022 JP 2022105474
(71) Applicant: Chemiteras, Inc., Yokohama-shi, Kanagawa 222-0033 (JP)
(72) Inventor: NAGAMATSU, Tomohisa, Kumamoto-shi, Kumamoto 860-0078 (JP); HIDE, Takuichiro, Yokohama-shi, Kanagawa 226-0018 (JP)
(74) Representative: Jones, Nicholas Andrew
(86) International application number: PCT/JP2023/024148
(87) International publication number: WO 2024/005131

(57) **Abstract**

An object of the present invention is to provide a drug that suppresses the proliferation of cancer stem cells. Accordingly, it is an object to provide a composition and a method that can treat cancer, suppress cancer recurrence and suppress cancer metastasis.

The above objects are achieved by a composition for suppressing proliferation of cancer stem cells comprising a triazolohalopyrimidine compound represented by the following general formula (I) as an active ingredient:

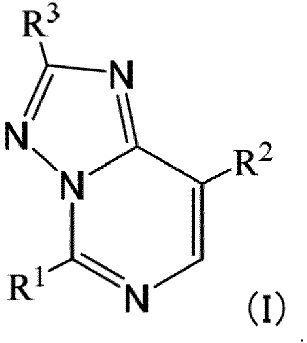

## Description

### Technical Field

The present inventions relate to a composition for suppressing proliferation of cancer stem cells and a method of suppressing proliferation of cancer stem cells

### Background Art

Cancer is the leading cause of death in Japan. Though many attempts to develop therapeutic method therefor are made, curing of cancer is still extremely difficult except for resectable cancer in initial stage.

It has been reported that in cancer, there are cell populations (so-called cancer stem cells) that have properties similar to those of normal stem cells, and that they get involved in the growth of cancer. In fact, complex tumor tissue is composed of a mixture of various stages of cancer cells from undifferentiated cells to highly differentiated cells. In other words, cancer stem cells present in tumor tissue constitute hierarchical tumor tissue by supplying "cancer cells" with various properties (cancer stem cell hypothesis). It has also been reported that cancer stem cells play important roles in stronger resistance to anticancer drugs and radiation than other cancer cells, and in cancer metastasis. It is considered to be a major factor in the recurrence of cancer despite the involution of cancer by existing anticancer agents and radiotherapy.

Therefore, there is a demand for a therapeutic drug that can not only cure cancer but also suppress recurrence and metastasis by inactivating all cancer cells through reliably inactivating cancer stem cells. There are great expectations for such drug, especially in brain tumors, where complete surgical resection of cancer is difficult.

However, there are only a few reports on compounds such as Dofequidar that are suggested to act on cancer stem cells (see Non-Patent Document 1), and no effective therapeutic agent is known yet.

On the other hand, the present inventors are conducting researches on triazolopyrimidine derivatives as compounds having antitumor activity (see Patent Document 1).

### Citation List

### Patent Documents

Patent Document 1:
WO2020/204024 A1

### Non-Patent Documents

Non-Patent Document 1:
Katayama R. et al., Cancer Science, Volume 100, Issue 11, p. 2060-2068, First published 11 October 2009

### Summary of the Invention

### Problems to be Solved by the Invention

An object of the present invention is to provide a medicine that suppresses the proliferation of cancer stem cells, and consequently to provide a composition and a method for capable of treating cancer, suppressing recurrence of cancer, and suppressing metastasis of cancer.

### Means of Solving the Problems

Accordingly, compositions and methods of one aspect of the present invention provide:
[1] A composition for suppressing proliferation of cancer stem cells comprising as a active ingredient triazolohalopyrimidine of the following general formula (I): 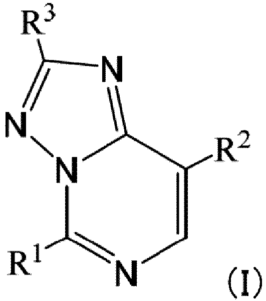 wherein R1 represents a chlorine atom or a bromine atom, R2 represents an alkyl group, and R3 an aryl group.
[2] Use of triazolohalopyrimidine of the general formula (I) above for production of a composition for suppressing proliferation of cancer stem cells.
[3] A process for suppressing proliferation of cancer stem cells which comprises bringing cancer stem cells into contact with triazolohalopyrimidine of the general formula (I) above.
[4] The composition according to [1], wherein the cancer stem cells are brain tumor stem cells.
[5] The composition according to [4], wherein the brain tumor stem cells is at least one type of the brain tumor stem cells selected from the group consisting of glioma stem cells and glioblastoma stem cells.
[6] The composition according to [1] or [4], wherein the composition is used for cure of cancer, suppression of recurrence of cancer and suppression of metastasis of cancer.
[7] The composition according to [1], wherein R¹ is a chlorine atom and/or R² is a methyl group.
[8] The composition according to [1] or [7], wherein R3 is a phenyl group or a substituted phenyl group.
[9] The composition according to [8], wherein the substituted phenyl group is a halogen substituted phenyl group.
[10] The composition according to [9], wherein the substituted phenyl group is selected from the group consisting of a bromophenyl group, a chlorophenyl group and a dichlorophenyl group.
[11] The composition according to [8], wherein the substituted phenyl group is selected from the group consisting of a hydroxyphenyl group, a nitrophenyl group and a cyanophenyl group.

### Effect of the Invention

According to the present invention, proliferation of cancer stem cells, which has been considered difficult due to drug resistance, can be suppressed. In addition, cancer stem cells are inactivated by suppressing the proliferation of cancer stem cells through loss of the self-replicating function and multipotency of cancer stem cells. As a result, the entire cancer inevitably become inactivated. That not only cures the cancer but also prevents its recurrence and metastasis to other sites.

### Brief Description of the Drawings

[Figure 1] It is a graph showing the evaluation results of the proliferation suppression effect on cancer stem cells, as shown in the Examples described below.
[Figure 2] It is a graph showing the evaluation results of the proliferation suppression effect on cancer stem cells, as shown in the Examples described below.

### Mode for carrying out the Invention

The details of each aspect of the present invention will be described below, but the technical scope of the present invention is not limited only by the matters of this item, and the present invention can take various forms as long as it achieves its purpose.

Each term used herein has the meaning commonly used by those skilled in the art, unless otherwise specified, and should not be construed as having an unduly restrictive meaning.

The term "content" as used herein has the same meaning as concentration, and means the ratio (eg mass %) of the amount (eg mass) of the component to the total amount (eg volume) of the agent. "Contains" means that it can add elements other than those explicitly included (which is synonymous with "including at least"), but includes "consisting of" and "consisting essentially of" . That is, "comprising" can mean including the specified element and any one or more elements, consisting of, or consisting essentially of the specified element.. Factors include limitations such as components, steps, conditions, parameters, and the like.

"~" in a numerical range is a range that includes the numerical values before and after it. For example, "0% to 100%" means a range of 0% or more and 100% or less. "greater than" and "less than" mean the lower and upper limits, respectively, excluding the preceding number; for example, "more than 1" means a numerical value larger than 1, and "less than 100" means a numerical value smaller than 100.

The number of digits of the integer value and the number of significant digits match. For example, 1 has 1 significant digit and 10 has 2 significant digits. Also, for decimal values, the number of digits after the decimal point and the number of significant digits are the same. For example, 0.1 has one significant digit and 0.10 has two significant digits.

Cancers targeted by the present invention include so-called cancers, malignant tumors, and sarcomas. Examples thereof include breast cancer such as hormone receptor-positive breast cancer, HER2-positive breast cancer, triple-negative breast cancer and hereditary breast cancer; brain tumor such as glioma (astrocytoma, oligodendroglioma, anaplastic astrocytoma, anaplastic oligoastrocytoma, anaplastic oligodendroglioma, glioblastoma, etc.), primary central nervous system lymphoma, meninglioma, pituitary adenoma, schwannoma and craniopharyngioma; prostate cancer; colorectal cancer such as adenocarcinoma, squamous cell carcinoma and adenosquamous carcinoma; head and neck cancer such as head and neck squamous cell carcinoma; and pancreatic cancer such as pancreatic ductal cancer and neuroendocrine cancer. The present invention can be preferably applied to cancers at sites where complete surgical resection is difficult, such as brain tumors, particularly highly malignant gliomas, and especially glioblastomas.

The term "cancer stem cell" in the present specification means cells among whole of cancer cells that retains self-replicating ability and retains differentiation ability under an appropriate environment, and it includes cancer stem-like cells as well. In addition, "cancer stem cells" are generally said to exhibit drug resistance to existing anticancer drugs.

It is believed that telomerase is expressed in stem cells, whereas normal cells lack telomerase. The same is true for cancer stem cells. Since normal somatic cells lack telomerase, their telomeres shorten with each cell division. Since telomerase is expressed in stem cells, the length of telomeres is supposed to be maintained.

The term "cancer cells" without any modifier in the present specification means a combination of cancer stem cells and other cancer cells, and has the same meaning as whole cancer cells. Other cancer cells have the same meaning as cancer cells other than cancer stem cells.

The term " suppression of proliferation of cancer stem cell" in the present specification means at least that the viable cell count or occupancy of cancer stem cells is not increased or is reduced compared to when the composition is not applied, and as a result, it includes inactivation of cancer stem cells. Inactivation of cancer stem cells means loss of substantial self-replicating ability and differentiation ability of cancer stem cells. It includes the loss of self-replicating ability and differentiation ability of cancer stem cells by causing cancer stem cells to die or by eliminating the cancer stem cells themselves.

Therefore, in the case of causing cell death of cancer stem cells, it can also be referred to as a composition for promoting cell death of cancer stem cells, and in the case of eliminating cancer stem cells, it can be referred to as a composition for removing cancer stem cells. In addition, since cancer stem cells have stronger resistance to anticancer drugs than other cancer cells, inactivating cancer stem cells inevitably lead to suppression of proliferation cancer cells.

A composition for suppressing proliferation of cancer stem cells according to one embodiment of the present invention is a composition containing a triazolohalopyrimidine compound represented by the general formula (I), hereinafter, it is also referred to as "triazolohalopyrimidine compound (I)", as an active ingredient.

The method of suppressing proliferation of cancer stem cells according to one embodiment of the present invention can be performed by bringing triazolohalopyrimidine compound (I) into contact with cancer stem cells. Since cancer stem cells cannot be distinguished from other cancer cells by visual observation or the like, and selective contact is practically impossible, in practice, it is brought into contact with cancer cells or cancer-affected area.

The triazolohalopyrimidine compound (I) can be brought into contact with cancer stem cells usually by administering the composition of one embodiment of the present invention for suppressing proliferation of cancer stem cells to patients or sick animals.

Specifically, the composition of one embodiment of the present invention for suppressing proliferation of cancer stem cells is applied orally, enterally, parenterally (intravenous injection, intravenous infusion), or locally applied (external application, injection into the affected area, placement in the affected area) and other routes.

The dosage is appropriately determined depending on the route of administration, patient's age, condition, body weight, etc. In the case of non-local application such as oral, enteral, parenteral (intravenous injection, intravenous infusion), the amount is usually 1 mg/kg body weight to 10 mg/kg body weight per day in terms of triazolohalopyrimidine compound (I). In the case of topical application, the dose can be selected from the range of 1 mg to 100 mg per 1 cm² of the area in contact with the affected area or 1 mg to 100 mg per 1 cm³ of the volume of the affected area.

Individuals who take the composition of one embodiment of the present invention for suppressing proliferation of cancer stem cells are not particularly limited, and examples thereof include animals, particularly mammals. Examples of mammals include humans, dogs, cats, cows, horses, pigs, sheep. and the like, and among these, humans are preferred.

The triazolohalopyrimidine compound (I) can be used alone in the composition for suppressing proliferation of cancer stem cells of the present invention, but usually it is preferred to apply a composition also containing a pharmaceutically acceptable carrier such as excipients and other additives.

Examples of carriers include solid carriers such as lactose, kaolin, sucrose, crystalline cellulose, cornstarch, talc, agar, pectin, magnesium stearate, lecithin, sodium chloride, and polypheprosan 20; and liquid carriers such as glycerol, peanut oil, polyvinylpyrrolidone, olive oil, ethanol, benzyl alcohol, propylene glycol and water.

The composition of one embodiment of the present invention for suppressing proliferation of cancer stem cells can take any form, that is, as a dosage form. Examples are tablets, powders, granules, and capsules when a solid carrier is used, and syrups, emulsions, creams, gels, pastes, and injections, when liquid carrier is used.

The active ingredient of the composition for suppressing proliferation of cancer stem cells of the present invention is the triazolohalopyrimidine compound (I) represented by the general formula (I).

R1 represents a chlorine atom or a bromine atom, a chlorine atom is preferred.

R2 represents an alkyl group such as a methyl group, an ethyl group, a propyl group, a butyl group, a pentyl group and a hexyl group, preferable examples of the alkyl group are C1 to C4 alkyl groups, more preferable example is a methyl group.

R3 represents an aryl group such as a phenyl group and a substituted phenyl group. Examples of the substituted phenyl groups include a halogen-substituted phenyl group such as a fluorophenyl group, a chlorophenyl group, a dichlorophenyl group, a trichlorophenyl group, a bromophenyl group, a dibromophenyl group, a tribromophenyl group, a iodophenyl group, a fluorochlorophenyl group and a bromochlorophenyl group; a hydroxy-substituted phenyl group such as a hydroxyphenyl group, a dihydroxyphenyl group and a trihydroxyphenyl group; an amino-substituted phenyl group such as an aminophenyl group, a N-methylaminophenyl group, a diaminophenyl group and triaminophenyl group; an alkoxy-substituted phenyl group such as a methoxyphenyl group, a dimethoxyphenl group, an ethoxyphenyl group, a propoxyphenyl group and a methylenedioxyphenyl group; a nitro-substituted phenyl group such as a nitrophenyl group and a dinitrophenyl group; and a cyano-substituted phenyl group such as a cyanophenyl group. Preferable examples thereof are a phenyl group, a fluorophenyl group, a chlorophenyl group, a bromophenyl group, a dichlorophenyl group, a difluorophenyl group, a dibromophenyl group, a hydroxyphenyl group, an aminophenyl group, a diaminophenyl group, a nitrophenyl group and a cyanophenyl group. A phenyl group, a fluorophenyl group, a chlorophenyl group a bromophenyl group, a dichlorophenyl group, a difluorophenyl group, a dibromophenyl group, a hydroxyphenyl group, an aminophenyl group, a nitrophenyl group and a cyanophenyl group are more preferable.

In a non-limiting embodiment of the present invention, the triazolohalopyrimidine compound (I) includes, for example, the one having a chlorine atom for R1, a methyl group for R2, and a halogen-substituted phenyl group, a hydroxyl-substituted phenyl group, or an amino-substituted phenyl group, a cyano-substituted phenyl group or an alkoxy-substituted phenyl group for R3. The compounds having chlorine atom for R1, a methyl group for R2, and a phenyl group, a chlorophenyl group, a bromophenyl group, a dichlorophenyl group, a dibromophenyl group, a hydroxyphenyl group, a dihydroxyphenyl group, a cyanophenyl group, a methoxyphenyl group or a methylenedioxyphenyl group for R3 are preferred.

Specific examples are the one in which R1 is a chlorine atom, R2 is a methyl group, and R3 is a phenyl group; the one in which R1 is a chlorine atom, R2 is a methyl group, and R3 is a chlorophenyl group; the one in which R1 is a chlorine atom, R2 is a methyl group, and R3 is a bromophenyl group; the one in which R1 is a chlorine atom, R2 is a methyl group, and R3 is a dichlorophenyl group; the one in which R1 is a chlorine atom, R2 is a methyl group, and R3 is a dibromophenyl group; the one in which R1 is a chlorine atom, R2 is a methyl group, and R3 is a hydroxyphenyl group; the one in which R1 is a chlorine atom, R2 is a methyl group, and R3 is a methoxyphenyl group; the one in which R1 is a chlorine atom, R2 is a methyl group, and R3 is a methylenedioxyphenyl group; the one in which R1 is a chlorine atom, R2 is a methyl group, and R3 is an aminophenyl group; the one in which R1 is a chlorine atom, R2 is a methyl group, and R3 is a cyanophenyl group; the one in which R1 is a bromine atom, R2 is a methyl group, and R3 is a phenyl group; the one in which R1 is a bromine atom, R2 is a methyl group, and R3 is a chlorophenyl group; the one in which R1 is a bromine atom, R2 is a methyl group, and R3 is a bromophenyl group; the one in which R1 is a bromine atom, R2 is a methyl group, and R3 is a dichlorophenyl group; the one in which R1 is a bromine atom, R2 is a methyl group, and R3 is a dibromophenyl group; the one in which R1 is a bromine atom, R2 is a methyl group, and R3 is a hydroxyphenyl group; the one in which R1 is a bromine atom, R2 is a methyl group, and R3 is a methoxyphenyl group; the one in which R1 is a bromine atom, R2 is a methyl group, and R3 is a methylenedioxyphenyl group; the one in which R1 is a bromine atom, R2 is a methyl group, and R3 is an aminophenyl group; and the one in which R1 is a bromine atom, R2 is a methyl group and R3 is a cyanophenyl group can be mentioned.

The triazolohalopyrimidine compound (I) may be in any form of free form, salt form or hydrate form (including hydrous salts). Examples of the salt form include inorganic acid salts such as hydrochlorides, sulfates and hydrobromides, organic acid salts such as oxalates, citrates and maleate, and ammonium salts. Pharmaceutically acceptable salts are particularly preferred.

Triazolohalopyrimidine compound (I) can be produced, for example, according to the scheme below, by the method described in WO2020-204024 (see Patent Document 1).

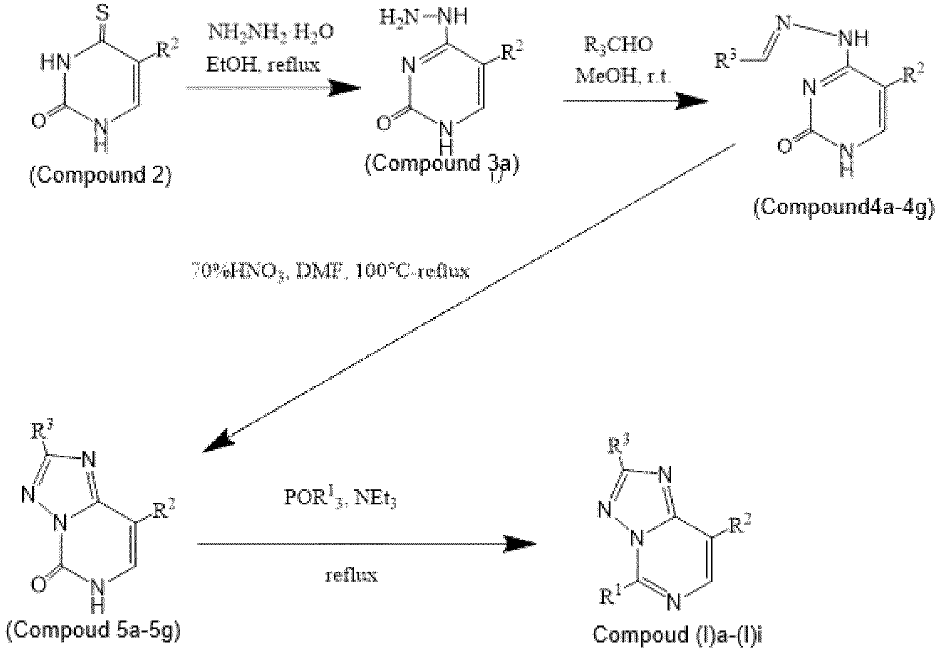

The present invention will be described in more detail below with reference to Examples, but the present invention is not limited to these Examples, and the present invention can take various aspects as long as the problems of the present invention can be solved.

### Examples

### [Example]

### [Manufacturing Example]

A mixture of 4-thiothymine (Compound 2) (1 g, 7.0 mmol) and hydrazine monohydrate (2 g, 40 mmol) in ethanol (8 ml) was heated under reflux for 10 minutes. Crystals precipitated after the reaction were collected by filtration. The obtained crystals were recrystallized with water, and colorless, needle crystals of 4-hydrazino-5-methylpyrimidin-2(1*H*)-one (compound 3a) (melting point (m.p.)> 300° C.) were obtained.

A mixture of 4-hydrazino-5-methylpyrimidin-2(1*H*)-one (compound 3a)(4 mmol) and benzaldehydes showed in Table 1 in methanol (25 ml) was stirred at room temperature for 0.5 to 2 hours. Crystals precipitated after the reaction were collected by filtration. The obtained crystals were recrystallized with ethanol, and 4-arylmethylidenhydrazino-5-methylpyrimidin-2(1*H*)-one (compound 4a-4g) was obtained in accordance with benzaldehydes used.

**[Table 1]**

| Used Compound | Used Benzaldehydes | Produced Compound |
|---|---|---|
| Compound 3a | benzaldehyde | Compound 4a |
| Compound 3a | 4-nitrobenzaldehyde | Compound 4b |
| Compound 3a | 3,4-dinitrobenzaldehyde | Compound 4c |
| Compound 3a | 4-bromobenzaldehyde | Compound 4d |
| Compound 3a | 4-cyanobenzaldehyde | Compound 4e |
| Compound 3a | 3,4-dichlorobenzaldehyde | Compound 4f |
| Compound 3a | 4-hydroxybenzaldehyde | Compound 4g |

A mixture of 4-arylmethylidenhydrazino-5-methylpyrimidin-2(1*H*)-one (compound 4a-4g)(1mmol) and 70%nitric acid (0.1 ml, 1.1mmol) in DMF(10 ml) was stirred under reflux for an hour. After the reaction, the solvent was removed under reduced pressure, the residue was treated with ethyl acetate, and precipitated solids were obtained by filtration. The precipitated solids were recrystallized by ethanol, and 2-aryl-8-methyl[1.2.4]triazolo[1.5-c]pyrimidin-5-(6*H*)-one (compound 5a-5g) corresponding to compound 4a to 4g was obtained.

A mixture of 2-aryl-8-methyl[1.2.4]triazolo[1.5-c]pyrimidin-5-(6*H*)-one (compound 5a-5g)(1.3 mmol) and triethylamine(4ml) in phosphorus oxychloride(10ml) was heated under reflux overnight. After the reaction, water was added little by little to the reaction mixture under ice-cooling to decompose unreacted phosphorus oxychloride in the reaction mixture. The reaction product was then subjected to extraction treatment using methylene chloride, and the solvent was distilled off under reduced pressure to precipitate crystals. A small amount of ethanol was added to the precipitated crystals, and the crystals were separated by filtration to obtain 2-aryl-5-chloro-8-methyl[1,2,4]triazolo[1,5-c]pyrimidine (triazolohalopyrimidine compound (I)a to (I)g) corresponding to compound 5a to 5g. Triazolohalopyrimidine compounds (I)a to (I)g have the structure of the following formula (I) (wherein R1 is a chlorine atom, R2 represents a methyl group, and R3 represents each functional group shown in Table 2), and their respective melting points are as shown in Table 2.

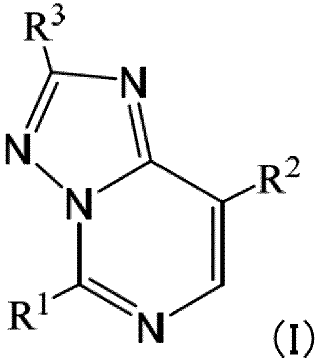

**[Table 2]**

| triazolohalopyrimidine compound | R³ | m.p. (°C) |
|---|---|---|
| (I)a | Phenyl | 151-155 |
| (I)b | 4-nitrophenyl | 166-168 |
| (I)c | 3,4-dinitrophenyl | 162-165 |
| (I)d | 4-bromophenyl | 165-168 |
| (I)e | 4-cyanophenyl | 245-248 |
| (I)f | 3,4-dichlorophenyl | >300 |
| (I)g | 4-hydroxyphenyl | 177-179 |

In the process obtaining Compound (I)a from Compound 5a, when the same operations are carried out as the operation obtaining Compound (I)a except substituting phosphorus oxybromide for phosphorus oxychloride, 2-phenyl-5-bromo-8-methyl[1.2.4]triazolo[1.5-c]pyrimidine (Compound (I)h) is obtained.

In the process obtaining Compound (I)d from Compound 5d, when the same operations are carried out as the operation obtaining Compound (I)d except substituting phosphorus oxybromide for phosphorus oxychloride, 2-(4-bromophenyl)-5-bromo-8-methyl[1.2.4]triazolo[1.5-c]pyrimidine (Compound (I)i is obtained.

### [Test Example 1]

The proliferation suppressive effect of the triazolohalopyrimidine compounds on cancer stem cells was evaluated as follows.

The proliferation suppressive effect of triazolohalopyrimidine compounds on cancer stem cells was evaluated using commercially available anticancer agents and known compounds known to have antitumor effect.

12037NS was used as cancer stem cells. 12037NS are the cells obtained by isolation and purification according to the method referred in Nature 432, No.18, 396-401 (2004). 12037NS cells are susceptible to cytotoxicity by DMSO (dimethylsulfoxide) used for dilution of test compounds. Accordingly, DMSO was used as control.

### (Cancer Stem Cells)

Used cancer stem cells were 12037NS which are cultivated cancer stem cells. 12037NS are the cells obtained by isolation and purification according to the method referred in Nature 432, No.18, 396-401 (2004).

Cancer stem cells (12037NS) were seededat 3.0×10³ cells/well in a 96-well plate using DMEM (Dulbeco's Modified Eagle Medium).

Test compounds shown in Table 3 were diluted with DMSO and then further diluted with PBS (phosphate buffered saline) to prepare test solutions. The test solution was added to each well to become a final concentration of 10 µM. For controls, DMSO was added to the wells to become a final concentration of 0.1% (v/v).

After adding the test solution, the plate was incubated at 37° C. in a 5%CO₂ environment for 3 days.

After incubation, water-soluble tetrazonium salt WST-8 ("Cell Counting Kit-8" coloring reagent; manufactured by Dojindo Laboratories) was added at 10 µL/well to each well and the plate was placed in a 37°C, 5%CO₂ environment, and incubated for 2 hours.

The absorbance at 450nm wavelength was measured for the culture solution in each well of the plate after incubation. WST-8 is reduced by intracellular dehydrogenase to produce water-soluble formazan. The number of living cells can be measured by measuring the absorbance of this formazan at 450nm.

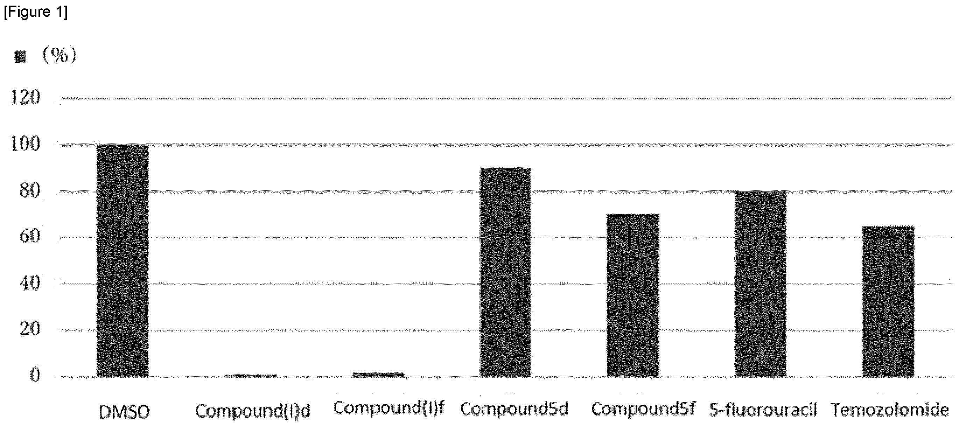

The survival rate of cancer stem cells was determined by comparing the measured number of living cells with the number of seeded cells. Table 3 and FIG. 1 show the survival rate of cancer stem cells for each test compound.

**[Table 3]**

| Experimental No. | Test Compound | Survival Rate(%) |
|---|---|---|
| Control 1 | DMSO | 100 |
| Example 1 | Triazolohalopyrimidine Compound (I)d | 1 |
| Example 2 | Triazolohalopyrimidine Compound (I)f | 2 |
| Comparative Example 1 | Triazolopyrimidine Compound 5d | 90 |
| Comparative Example 2 | Triazolopyrimidine Compound 5f | 70 |
| Comparative Example 3 | 5-Fluorouracil | 80 |
| Comparative Example 4 | Temozolomide | 65 |

In Table 3, the meaning of each test compound is as follows:
DMSO: Control
Triazolohalopyrimidine Compound (I)d: 2-(4-bromophenyl)-5-chloro-8-methyl[1.2.4]triazolo[1,5-*c*]pyrimidine
Triazolohalopyrimidine Compound (I)f: 2-(3,4-dichlorophenyl)-5-chloro-8-methyl[1.2.4]triazolo[1,5-*c*]pyrimidine
Triazolohalopyrimidine Compound (I)g: 2-(4-hydroxyphenyl)-5-chloro-8-methyl[1.2.4]triazolo[1,5-*c*]pyrimidine
Triazolopyrimidine Compound 5d: 2-(4-bromophenyl)-8-methyl[1.2.4]triazolo[1,5-c]pyrimidin-5(6*H*)-on
Triazolopyrimidine Compound 5f: 2-(3,4-dichlorophenyl)-8-methyl[1.2.4]triazolo[1,5-c]pyrimidin-5(6*H*)-on
5-fluorouracil: commercially available anticancer agent
Temozolomide: commercially available anticancer agent

As shown in Table 3 and FIG. 1, triazolopyrimidine compounds 5d and 5f had cancer stem cell survival rate of at least 70%, whereas the corresponding triazolopyrimidine compounds (I)d and (I)f was less than 2%.

Furthermore, the proliferation suppressive effect of the triazolohalopyrimidine compounds (I)d and (I)f on cancer stem were significantly superior to those of commercially available anticancer agents.

### [Test Example 2]

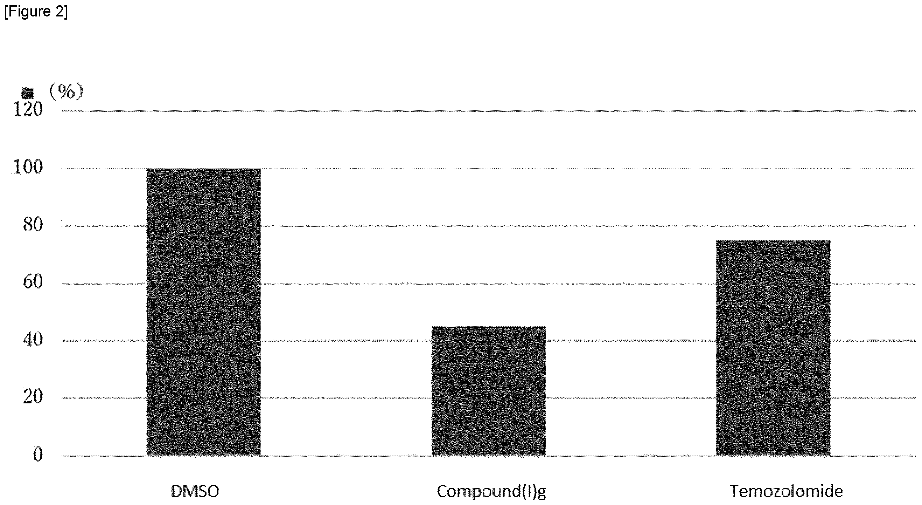

In the same manner as in [Test Example 1], the survival rate of cancer stem cells was determined for the test compounds shown in Table 4. Table 4 and FIG. 2 show the survival rate of cancer stem cells for each test compound. Triazolohalopyrimidine compound (I)g means 2-(4-hydroxyphenyl)-5-chloro-8-methyl[1.2.4]triazolo[1,5-*c*]pyrimidine.

**[Table 4]**

| Experimental No. | Test Example | Survival Rate (%) |
|---|---|---|
| Control 2 | DMSO | 100 |
| Example 3 | Triazolohalopyrimidine Compound (I)g | 45 |
| Comparative Example 5 | Temozolomide | 75 |

As shown in Table 4 and FIG. 2, the triazolohalopyrimidine compound (I) g had a cancer stem cell survival rate of 45%, and exhibited an excellent proliferation suppressive effect on cancer stem cells.

As described above, it was found that the triazolohalopyrimidine compound (I) in one embodiment of the present invention exhibits proliferation suppressive effect on cancer stem cells.

### Industrial applicability

According to the present invention, by using the triazolohalopyrimidine compound represented by the general formula (I) as an active ingredient, the proliferation of cancer stem cells can be suppressed. It can treat cancer, suppress cancer recurrence and suppress cancer metastasis, and is particularly applicable to cancers at sites where complete surgical resection is difficult, such as brain tumors, especially highly malignant gliomas, particularly glioblastomas. Therefore, it is useful for the ingesting individuals and can be used as a pharmaceutical or the like that contributes to the health and welfare of such ingesting individuals.

## Claims

1. A composition for suppressing proliferation of cancer stem cells containing as an active ingredient a triazorohalopyrimidine compound represented by the following formula (I): 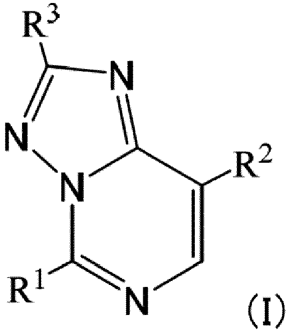 wherein R1 represents a chlorine atom or a bromine atom, R2 represents an alkyl group, and R3 represents an aryl group.

2. The composition according to Claim 1, wherein the cancer stem cells are brain tumor stem cells.

3. The composition according to Claim 2, wherein the brain tumor stem cells are at least one brain tumor stem cells selected from the group consisting of glioma stem cells and glioblastoma stem cells.

4. The composition according to Claim 1, wherein the compound is used for treatment of cancer, suppression of recrudescence of cancer and suppression of metastasis of cancer.

5. The composition according to Claim 1, wherein the R1 is a chlorine atom and/or the R2 is a methyl group.

6. The composition according to Claim 1 or 5, wherein R3 is a phenyl group or a substituted phenyl group.

7. The composition according to Claim 6, wherein the substituted phenyl group is a halogen substituted phenyl group.

8. The composition according to Claim 7, wherein the substituted phenyl group is the one selected from the group consisting of a bromophenyl group, a chlorophenyl group and a dichlorophenyl group.

9. The composition according to Claim 6, wherein the substituted phenyl group is the one selected from the group consisting of a hydroxyphenyl group, a nitrophenyl group and a cyanophenyl group.
